(19) Europäisches Patentamt
European Patent Office
Office européen des brevets

(11) EP 0 454 058 B1

(12) EUROPÄISCHE PATENTSCHRIFT

(45) Veröffentlichungstag und Bekanntmachung des Hinweises auf die Patenterteilung:
**17.06.1998 Patentblatt 1998/25**

(51) Int. Cl.$^6$: **A61K 31/12**, A61K 31/185

(21) Anmeldenummer: **91106517.5**

(22) Anmeldetag: **23.04.1991**

# (54) Verwendung von Anthrachinonderivaten zur Prophylaxe und Therapie von Viruserkrankungen

Use of anthraquinone derivatives in the prophylaxis and treatment of viral diseases

Utilisation de dérivés d'anthraquinone dans la prophylaxie et le traitement de maladies virales

(84) Benannte Vertragsstaaten:
**AT BE CH DE DK ES FR GB GR IT LI LU NL SE**

(30) Priorität: **24.04.1990 DE 4013023**

(43) Veröffentlichungstag der Anmeldung:
**30.10.1991 Patentblatt 1991/44**

(73) Patentinhaber:
**LOMAPHARM**
**Rudolf Lohmann GmbH KG**
**Pharmazeutische Fabrik**
**31857 Emmerthal (DE)**

(72) Erfinder:
- **May, Gerhard, Prof. Dr. med.**
  **W-6380 Bad Homburg (DE)**
- **Leonhadt, Klaus, Dr.**
  **W-3251 Ottenstein (DE)**
- **Ott, Holger**
  **W-3254 Emmerthal 1 (DE)**

(74) Vertreter:
**Schwabe - Sandmair - Marx**
**Stuntzstrasse 16**
**81677 München (DE)**

(56) Entgegenhaltungen:
**WO-A-89/05141** **DE-A- 3 913 040**
**US-A- 3 838 177** **US-A- 3 974 186**
**US-A- 4 670 265**

- **PATENT ABSTRACTS OF JAPAN, Band 13, Nr. 362 (C-625)[3710], 14. August 1989; & JP-A-1 121 215**
- **JOURNAL OF NATURAL PRODUCTS, Band 52, Nr. 5, September-Oktober 1989, Seiten 987-995; T. KONOSHIMA et al.: "Studies on inhibitors of skin tumor promotion. VI. Inhibitory effects of quinones on epstein-barr virus activation"**
- **BIOCHEMICAL AND BIOPHYSICAL RESEARCH COMMUNICATIONS, Band 130, Nr. 1, 16. Juli 1985, Seiten 249-256; R.T. SU et al.: "Inhibition of initiation of simian virus 40 chromosome synthesis by dihydroxyanthraquinone"**
- **JOURNAL OF MEDICINAL CHEMISTRY, Band 17, Nr. 19, 1974, Seiten 965-968; A.D. SILL et al.: "Bis-basic-substituted polycyclic aromatic compounds. A new class of antiviral agents. 1,2 5. Bis-basic ethers of anthraquinone and bisalkamine esters of anthraquinonedicarboxylic acids"**
- **ANTIVIRAL RESEARCH, Band 13, Nr. 5, Mai 1990, Seiten 265-272; R.F. SCHINAZI et al.: "Anthraquinones as a new class of antiviral agents against human immunodeficiency virus"**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 454 058 B1

**Beschreibung**

Die Erfindung betrifft die Verwendung bestimmter Anthrachinonderivate zur Prophylaxe und Therapie von Viruserkrankungen zur Herstellung eines Arzneimittels mit einem Gehalt an bestimmten Anthrachinonderivaten zur Behandlung von speziellen Viruserkrankungen wie in den Ansprüchen im einzelnen gekennzeichnet.

Anthrachinone stellen die größte Gruppe natürlich vorkommender Chinone dar. Fast alle natürlich vorkommenden Anthrachinone sind hydroxyliert. Viele Vertreter dieser Naturstoffe wurden aus niederen Pilzen, ein weiterer, größerer Teil, aus Pflanzen isoliert. Eines der bekanntesten Anthrachinonderivate, das Alizarin (1,2-Dihydroxy-9,10-anthrachinon), kommt in den Wurzeln der Pflanze Rubia tinctorum glykosidisch gebunden an das Disaccharid Primverose als Ruberythrinsäure vor. Durch enzymatische Hydrolyse entsteht während der Trocknung der Krappwurzel aus der Ruberythrinsäure der rote Farbstoff Alizarin. Das Alizarin wurde als Farbstoff schon im Altertum eingesetzt. Seit 1869 (Caro et al., 1870 (1)) kann Alizarin chemisch synthetisiert werden.

Für die Droge der Krappwurzel wurden diuretische (Formanek und Rácz-Kotilla, 1972 (2)) und harnsteinauflösende (Formanek et al., 1973 (3)) Wirkungen in vitro beschrieben, die vor allem der Ruberythrinsäure zugeordnet werden. Beide Wirkungen werden jedoch inzwischen bezweifelt. Dagegen ist eine mäßige spasmolytische Wirkung gesichert. Bestimmte Anthrachinonderivate werden als Abführmittel verwendet.

Die DE-A-3 913 040 betrifft die Verwendungen von Konjugaten von Albumin mit Komplexen aus jeweils zwei verschiedenen Anthrachinonverbindungen, die stets zusammen mit einer Pilzkultur zu Arzneimitteln zur Behandlung von beispielsweise Retrovireninfektionen verarbeitet werden.

Die Lehre gemäß der US-A-4 670 265 bedient sich des Einsatzes von Pflanzenextrakten aus Aloe, Rhamnus, Cassia oder Rheum; insbesondere wird darin auch das Aloe-Emodin zur Behandlung von Virusinfektionen erwähnt.

Die WO-A-8905141 befaßt sich insbesondere mit verschiedenen Flavonen, aber auch mit Alizarin als Mittel gegen retrovirusbedingte Infektionen.

Im J. Med. Chem., Bd. 17, Nr. 9 (1974), S. 965-968 sind dibasisch substituierte Anthrachinonverbindungen beschrieben, die in Form von Estern oder Ethern bzw. Aminosulfonsäuresalzen vorliegen können. Die Verwendung dieser Verbindungen bezieht sich speziell auf den Enzephalomyokarditis-Virus (GMC), aber auch auf Myxo-Viren (Influenza A), den Arbo-Virus Semliki Forest, oder den Stomatitis-Virus.

Die US-A-3 974 186 betrifft die Behandlung von unter anderem Herpes-Viren und beschreibt für diesen Indikationdszweck ganz speziell substituierte 2,6- und 2,7-Dihydroxyanthrachinone.

Die Anmelderin hat nun überraschend gefunden, daß ganz speziell substituierte Anthrachinonderivate der folgenden allgemeinen Formen (I) eine virustatische Wirkung gegen Viren der Familie Herpetoviridae zeigen:

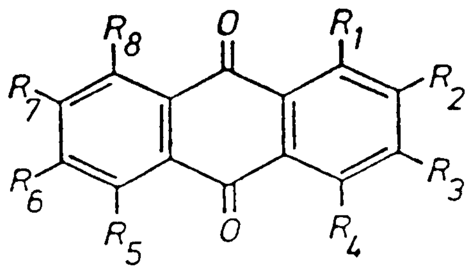

I

in der $R_1$ bis $R_8$ H, OH, $C_1$-Alkyl, $C_2$-Alkyl, $C_3$-Alkyl, $C_4$-Alkyl, $NH_2$, COOH, $SO_3$, $CH_2OH$ oder $C_6H_{11}O_5$ bedeuten, wobei nicht jeder der Reste R1 bis R8 die gleiche Bedeutung aufweist, und die pharmazeutisch annehmbaren Salze, Ester und Ether, mit Ausnahme von Aloe-Emodin und anderen Anthrachinone enthaltenden Pflanzen extrakten aus Aloe vera, Rhamnus frangula, Rhamnus purshiana, Cassia angustifolia und Rheum rhaponticum sowie von 2,6- bzw. 2,7-Bis-(gegebenenfalls halogenierten)-alkylaminoalkoxyanthrachinonen und deren sauren Additionssalzen.

Eine bevorzugte Gruppe von Verbindungen ist jene, in der

$R_1$ : H, OH, $CH_3$, $NH_2$
$R_2$ : H, OH, $CH_3$, $CH_2OH$, COOH, $C_2H_5$, $NH_2$, $SO_3$
$R_3$ : H, OH, $CH_3$
$R_4$ : H, OH, $NH_2$
$R_5$ : H, OH, $HN_2$

$R_6$ : H, OH, $CH_3$
$R_7$ : H, OH, $C_6H_{11}O_5$ und
$R_8$ : H, OH, $NH_2$ bedeuten.

Von den Verbindungen der Formel (I) wurden als besonders wirksam die folgenden Verbindungen gefunden: Alizarin (1: 1,2-Dihydroxy-9,10-anthracendion, 1,2-Dihydroxyanthrachinon), Alizarinsulfonsäure (2: 9,10-Dihydro-3,4-dihydroxy-9,10-dioxo-2-anthracensulfonsäure.Mononatriumsalz), Chinizarin (3: 1,4-Dihydroxyanthrachinon), Chrysazin (4: Danthron, 1,8-Dihydroxyanthrachinon) und Anthraflavinsäure (5: 2,6-Dihydroxyanthrachinon). Die erwähnten Verbindungen haben die folgenden Strukturformeln:

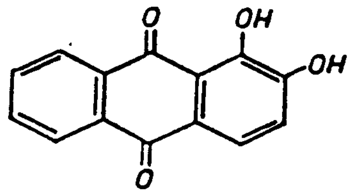

1

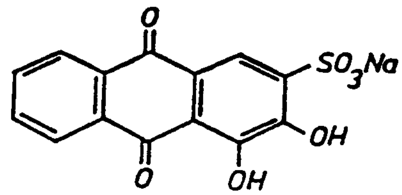

2

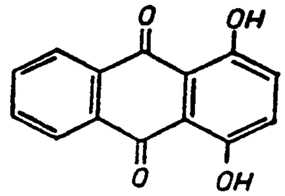

3

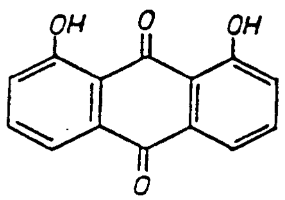

4

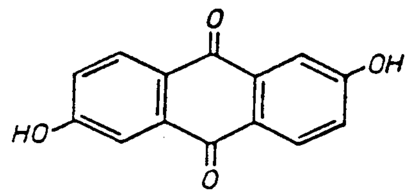

5

Die Verbindungen der Formel (I) können nach bekannten Verfahren hergestellt werden (vgl. z. B. Beyer und Walter, Lehrbuch der organischen Chemie, 21. Auflage, Seiten 639 bis 643, Hirzel-Verlag, Stuttgart, 1988). Verschiedene Anthrachinonderivate der Formel (I) sind auch kommerziell erhältlich.

Unter der Bezeichnung $C_1$-Alkyl, $C_2$-Alkyl, $C_3$-Alkyl und $C_4$-Alkyl sind Alkylgruppen mit 1, 2, 3 oder 4 C-Atomen mit gerader oder verzweigter Kette zu verstehen.

Die pharmazeutisch annehmbaren Salze können Säureadditionssalze oder Ammonium-, Alkalimetall- oder Erdalkalimetallsalze sein.

Geeignete Ester und Ether umfassen Verbindungen mit $C_1$- bis $C_4$-Alkylgruppen, worin die Alkylgruppe eine gerade oder verzweigte Kette ist.

Die Verbindungen können zur Herstellung jeder der im Stand der Technik bekannten pharmazeutischen Formulierung verwendet werden.

Die virustatische Wirkung zeigt sich insbesondere gegen Viren der Familie Herpetoviridae, Z. B. HSV (Herpes simplex-Virus), CMV (Cytomegalie-Virus) und HHV 6 (Humanes Herpes-Virus Typ 6). Zur Familie der Herpetoviridae rechnet man weiterhin das Varicella-Zoster-Virus und das Epstein-Barr-Virus.

Im folgenden wird die virustatische Wirkung der Verbindungen der Formel (I) näher beschrieben.

**Methoden zur Bestimmung der antiviralen Aktivität**

Die Prüfung der virustatischen Wirkung erfolgt in vitro mit virusinfizierten tierischen Zellkulturen. Entsprechend der Eigenschaften der eingesetzten Testviren müssen unterschiedliche Testvarianten und Wirtszellen für die Untersuchungen ausgewählt werden.

**1. Farbtest**

Der Farbtest, wie von Armstrong, 1971 (5) beschrieben, beruht darauf, daß die durch die Viren zerstörten Zellen nicht mehr gefärbt werden können. Je höher der Anteil an intakten Zellen nach der Inkubation mit dem jeweiligen Virus durch die virustatische Wirkung einer Substanz ist, um so mehr Farbstoff wird gebunden. Der Farbstoff läßt sich aus den gefärbten Zellen extrahieren und seine Menge kann in einem Photometer, z. B. bei Verwendung von Kristallviolett bei 546 nm, gemessen werden. Damit erlaubt der Test meßbare und reproduzierbare Angaben sowohl über die virustatische, wie auch durch Parallelversuche mit Zellkulturen ohne Viren über die cytotoxische Wirkung von Substanzen.

Durchführung: 10 mg der Testsubstanz wurden in 10 ml Ethylalkohol gelöst. Unter Verwendung des Mediums für die Zellkultur wurde eine Verdünnungsreihe mit Konzentrationen von 1,56 µg/ml bis 25 µg/ml Testsubstanz eingestellt. Diese Lösungen werden auf 24 Stunden alte MK2-, HeLa-, Rita-Zellen oder andere geeignete Zellen gegeben, nachdem diese vorher 45 Minuten zur Infektion mit einer Suspension von Viren überschichtet worden waren. Zur Prüfung der Cytotoxizität werden Parallelansätze ohne Viruszugabe analog mit den verschiedenen Verdünnungsstufen der Prüfsubstanz überschichtet. Die Versuchsansätze mit Virus und Testsubstanz bzw. nur der Testsubstanz werden 72 Stunden bei 37 °C bebrütet. Anschließend werden die Zellen fixiert und für 15 Minuten mit Kristallviolettlösung gefärbt. Nach Spülen mit Wasser wird der an die Zellen gebundene Farbstoff mit Ethanol extrahiert und in einem Photometer die Extinktion bei 546 nm (Absorption für Kristallviolett) gemessen.

Zur Auswertung werden die Extinktionen und auch die Konzentration mit einem Zellschutz von 50 % berechnet.

Falls die Testsubstanz keine wesentlichen cytotoxischen Eigenschaft besitzt, kann die antivirale Schutzwirkung nach nach folgender Formel in Prozent berechnet werden:

$$\frac{E_3 - E_2}{E_1 - E_2} \times 100$$

$E_1$ = Extinktion der Zellkontrolle
$E_2$ = Extinktion der Viruskontrolle
$E_3$ = Extinktion der Versuchsansätze mit Virus und Alizarin

**2. Plaquehemmungstest**

Der Plaquehemmungstest (Agardiffusionstest) wird nach der Methode von E. C. Herrmann et al., 1960 (6) durchgeführt. Auch bei diesem Test werden virusinfizierte tierische Zellkulturen verwendet. In diesem Fall wird in Petrischalen durch ein mit Hilfe von Agar verfestigtes Medium das unkontrollierte Ausbreiten der Viren verhindert. Es kommt zu einer lokal begrenzten Zerstörung der Zellen, die nach einer Färbung als Löcher im Zellrasen erkennbar ist. Die Testsubstanzen werden auf Filterpapierblättchen aufgetragen und auf das feste Medium aufgelegt. In einem Bereich um diese Blättchen werden durch die virustatische Wirkung der Testsubstanz die Zellen geschützt und es entstehen so kreisförmige Hemmzonen. Die Größe der Hemmzone kann als Maß für die virustatische Wirksamkeit ausgewertet werden.

Durchführung: Ein Monolayer von MK2-, HeLa-, Rita-Zellen oder einer anderen geeigneten Zellinie wird in einer Petrischale mit Viren infiziert, so daß eine große Zahl von einzelnen Plaques entsteht. Nach einer Bindung von 45 Minuten bei Zimmertemperatur wird der Zellrasen einmal mit phosphatgepufferter Kochsalzlösung gewaschen und mit einer Agar-Oberschicht versehen. Auf diese Agarschicht werden Filterpapierblättchen (10 mm Durchmesser) gelegt, die mit einer Lösung der Testsubstanz getränkt sind. Nach 48 bis 72 Stunden Bebrütung bei 37 °C in $CO_2$-Atmosphäre (5 %) wird eine zweite Oberschicht darübergegeben, die den Vitalfarbstoff Tetrazoliumchlorid enthält. Nach weiteren 24 Stunden Bebrütung wird der Hemmhofdurchmesser, unterteilt in "toxische Zone" und "therapeutische Zone", in mm ausgemessen.

**3. Plaquereduktionstest**

Für die Prüfung der Wirksamkeit gegenüber Cytomegalie-Viren (CMV) werden mit diesen Viren infizierte menschliche Vorhautfibroblasten eingesetzt. Nach der Inkubation werden die entstandenen Plaques angefärbt und ausgezählt. Zur Auswertung wird die Plaquezahl unter dem Einfluß einer definierten Substanzmenge mit der Viruskontrolle verglichen.

Die Ergebnisse der Untersuchungen mit verschiedenen Anthrachinonderivaten werden im folgenden beschrieben:

**Wirkung des Alizarins (1) auf die Vermehrung von HSV**

Die Ergebnisse der Untersuchungen mit Alizarin (1) im Farbtest gegen Herpes simplex-Viren sind in den Tabelle Ia und I b wiedergegeben. Im Vergleich zur Viruskontrolle, d. h. virusinfizierten Ansätzen ohne Testsubstanz, sind deutlich höhere Extinktionswerte (siehe Tabelle I a) und damit eine antivirale Wirkung des Alizarins (1) erkennbar.

Tabelle Ia

| Bestimmung der virustatischen Wirkung des Alizarins (1) gegen Herpes simplex-Viren in Rita-Zellkulturen | | |
|---|---|---|
| Alizarin (µg/ml) | Extinktion bei 546 nm | |
| | Hela-Zellen | Hela-Zellen mit Herpes-Virus |
| 25 | 0,328 | 0,235 |
| 12,5 | 0,510 | 0,338 |
| 6,25 | 0,634 | 0,403 |
| 3,125 | 0,693 | 0,329 |
| 1,56 | 0,655 | 0,206 |
| ZK | 0,648 | |
| VK | | 0,144 |
| ZK = Zellkontrolle<br>VK = Viruskontrolle | | |

Die Extinktion mit 50 %-Zellschutz (ZK + VK)/2 liegt bei 0,4. Eine Konzentration von 5,96 µg/ml Alizarin bewirkt einen Zellschutz von 50 % (Ergebnisse siehe Tabelle I a).

Weiterhin liegt der Mittelwert für eine 50 %ige Hemmung der Virusvermehrung aus vier Versuchen bei 4,87 µg/ml Alizarin.

Tabelle I b

| Zellschutz (%) des Alizarins in Hela-Zellkulturen gegen Herpes simplex-Viren | | | | | |
|---|---|---|---|---|---|
| | Alizarin (µg/ml) | | | | |
| | 25 | 12,5 | 6,25 | 3,125 | 1,56 |
| Zellschutz (%) | 18 | 38 | 51 | 37 | 12 |

Die Abnahme der Schutzwirkung (Ergebnisse siehe Tabelle I b) bei den höheren Konzentrationen wird durch eine schwache cytotoxische Wirkung verursacht, die bei diesen Konzentrationen die virustatische Wirkung überlagert (siehe Tabelle I a und I b).

In Gegenwart von 10 % Serum, das als Beispiel für ein Fremdprotein zugegeben wurde, ist der 50 % Hemmwert geringfügig höher bei 11,3 µg/ml. Daran ist zu erkennen, daß Alizarin auch in Gegenwart von Fremdproteinen eine sehr gute antivirale Wirkung hat.

**Wirkung des Alizarins auf die Vermehrung von CMV**

Mit einer ähnlichen Versuchsanordnung wie im Plaquehemmungstest kann die Wirkung des Alizarins gegen Cytomegalie-Viren bestimmt werden (siehe Plaquereduktionstest, Methode 3). Die Untersuchung für das Cytomegalie-Virus in einer Zellinie von menschlichen Vorhautfibroblasten ergab die in Tabelle II angegebenen Werte für die Hemmung im Vergleich zur Viruskontrolle. Zur Auswertung wurden hier die gebildeten Plaques gezählt und im Vergleich zur Viruskontrolle ausgewertet (siehe Tabelle II).

Tabelle II

| Hemmung (%) der Vermehrung von Cytomegalie-Viren in Kulturen von menschlichen Vorhautfibroblasten im Plaquereduktionstest | | | | |
|---|---|---|---|---|
| | Alizarin (µg/ml) | | | |
| | 20 | 15 | 10 | 5 |
| Hemmung (%) | 100 | 100 | 60 | 10 |

**Wirkung des Alizarins auf die Vermehrung von HHV 6**

In weiteren Versuchen wurde die Wirkung von Alizarin auf die Vermehrung von HHV 6 (Human herpes-Virus Typ 6) untersucht. Diese Viren lassen sich aus dem Blut von immunsupprimierten Patienten mit Leukämie, Lymphomen oder Aids sowie von Kindern mit Roseola infantum isolieren.

Die Testung erfolgte in menschlichen Lymphocytenkulturen.

In den Zellen war schon nach drei Passagen in Gegenwart von 10 µg/ml Alizarin kein Virus mehr nachweisbar, so daß im Gegensatz zur Viruskontrolle keine cytopathischen Veränderungen erkennbar sind.

Die vorstehenden Ergebnisse zeigen, daß Alizarin über ausgezeichnete virustatische Eigenschaften verfügt, die seine Verwendung in der Therapie und Prophylaxe, insbesondere für Herpes- und Cytomegalie-Virusinfektionen, als vorteilhaft erkennen lassen.

Im folgenden wird beispielhaft die virustatische Wirkung von weiteren Anthrachinonderivaten gezeigt.

Neben dem Alizarin zeigen auch andere Anthrachinonderivate eine gute virustatische Wirksamkeit. Die Ergebnisse der Untersuchungen mit diesen Substanzen im Plaquehemmungstest mit Herpes simplex-Viren in Rita-Zellen sind in der Tabelle III zusammengefaßt.

Tabelle III

| Ergebnisse der Prüfung des Alizarins und weiterer Anthrachinonderivate im Plaquehemmungstest gegen Herpes simplex-Viren | | | |
|---|---|---|---|
| Testsubstanz | Hemmhofdurchmesser (mm) Substanzmenge (µg/ml) pro Testblättchen | | |
| | 10 | 20 | 40 |
| Alizarin (1) | 14 | 18 | 20 |
| Alizarinsulfonsäure (2) | 10 | 12 | 19 |
| Chinizarin (3) | 11 | 13 | 16 |
| Chrysazin (4) | 8 | 12 | 16 |
| Anthraflavinsäure (5) | 9 | 13 | 15 |

Die beschriebenen Eigenschaften eröffnen aussichtsreiche Möglichkeiten für den Einsatz dieser Verbindungen in der Prophylaxe und Therapie von Viruserkrankungen, insbesondere bei Infektionen mit Viren der Herpes-Gruppe.

**Literaturverzeichnis:**

(1) Caro et al., Ber. 3, 359 (1870)

(2) Formanek, I und Rácz-Kotilla E., Farmacia (Bucuresti) 7, 439 (1972)

(3) Formanek, I., Rácz-Kotilla E. und Sebe A., Rev. Med. (Tirgu-Mures) 17, 427 (1971)

(4) Beyer und Walter, Lehrbuch für organische Chemie, 21. Auflage, S. Hirzel-Verlag, Stuttgart, 1988, Seiten 639

bis 643

(5) Armstrong, J. A., Semi-micor, dye-binding assay for rabbit interferon, Appl. Microbiol. 21, 723 bis 7256 (1971)

(6) Hermann, E. C., Gabliks, J., Engle, C. And Perlman, P. L., Agar diffusion method for detection and bioassay of antiviral antibiotics, Proc. Soc. Exp. Biol. Med., N. Y. 103, 625 bis 628 (1960)

**Patentansprüche**

1. Verwendung einer Verbindung der allgemeinen Formel (I) zur Herstellung eines Medikaments für die Prophylaxe und/oder Therapie von Erkrankungen, bedingt durch ein Virus der Familie Herpetoviridae

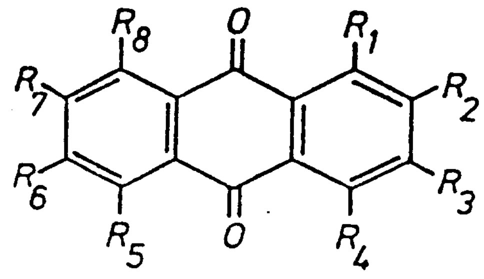

I

in der
$R_1$ bis $R_8$, H, OH, $C_1$-Alkyl, $C_2$-Alkyl, $C_3$-Alkyl, $C_4$-Alkyl, $NH_2$, COOH, $SO_3$, $CH_2OH$ oder $C_6H_{11}O_5$ bedeuten, wobei nicht jeder der Reste $R_1$ bis $R_8$ die gleiche Bedeutung aufweist, und die pharmazeutisch annehmbaren Salze, Ester und Ether, mit Ausnahme von Aloe-Emodin und anderen Anthrachinone enthaltenden Pflanzenextrakten aus Aloe vera, Rhamnus frangula, Rhamnus purshiana, Cassia augustifolia und Rheum rhaponticum sowie von 2,6- bzw. 2,7-Bis-(gegebenenfalls halogenierten)-alkylaminoalkoxyanthrachinonen und deren sauren Additionssalzen.

2. Verwendung nach Anspruch 1 mit mindestens einer Verbindung, worin die Reste $R_1$ bis $R_8$ in der allgemeinen Formel (I) bedeuten:

$R_1$ : H, OH, $CH_3$, $NH_2$
$R_2$ : H, OH, $CH_3$, $CH_2OH$, COOH, $C_2H_5$, $NH_2$, $SO_3$
$R_3$ : H, OH, $CH_3$
$R_4$ : H, OH, $NH_2$
$R_5$ : H, OH, $HN_2$
$R_6$ : H, OH, $CH_3$
$R_7$ : H, OH, $C_6H_{11}O_5$ und
$R_8$ : H, OH, $NH_2$.

3. Verwendung nach Anspruch 1 oder 2 mit mindestens einer der folgenden Verbindungen: 1,2-Dihydroxy-9,10-anthracendion, 9,10-Dihydro-3,4-dihydroxy-9,10-dioxo-2-anthracensulfonsäure.Mononatriumsalz, 1,4-Dihydroxyanthrachinon, 1,8-Dihydroxyanthrachinon und 2,6-Dihydroxyanthrachinon.

4. Verwendung nach Anspruch 3, bei der das Virus ein Herpes simplex-Virus (HSV), Cytomegalie-Virus (CMV) oder ein Herpesvirus vom Typ 6 (HHV 6) ist.

**Claims**

1. Use of a compound of the general formula (I) for the preparation of a medicament for the prophylaxis and/or therapy of diseases caused by a virus of the Herpetoviridae family

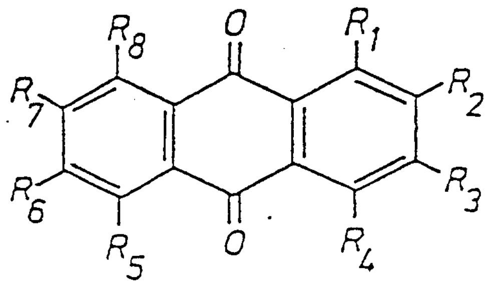

(I)

I

in which
$R_1$ to $R_8$ denote H, OH, $C_1$ alkyl, $C_2$ alkyl, $C_3$ alkyl, $C_4$ alkyl, $NH_2$, COOH, $SO_3$, $CH_2OH$ or $C_6H_{11}O_5$, with not every radical $R_1$ to $R_8$ denoting the same, and the pharmaceutically acceptable salts, esters and ethers, with the exception of aloe-emodine and other anthraquinone-containing plant extracts of Aloe vera, Rhamnus frangula, Rhamnus purshiana, Cassia angustifolia and Rheum rhaponticum and of 2,6- or 2,7-bis(optionally halogenated)alkylamino alkoxyanthraquinones and acid addition salts thereof.

2. Use according to Claim 1 having at least one compound in which the radicals $R_1$ to $R_8$ in the general formula (I) denote:

$R_1$ : H, OH, $CH_3$, $NH_2$
$R_2$ : H, OH, $CH_3$, $CH_2OH$, COOH, $C_2H_5$, $NH_2$, $SO_3$
$R_3$ : H, OH, $CH_3$
$R_4$ : H, OH, $NH_2$
$R_5$ : H, OH, $NH_2$
$R_6$ : H, OH, $CH_3$
$R_7$ : H, OH, $C_6H_{11}O_5$ and
$R_8$ : H, OH, $NH_2$.

3. Use according to Claim 1 or 2 having at least one of the following compounds: 1,2-dihydroxy-9,10-anthracenedione, monosodium salt of 9,10-dihydro-3,4-dihydroxy-9,10-dioxo-2-anthracene sulphonic acid, 1,4-dihydroxyanthraquinone, 1,8-dihydroxyanthraquinone and 2,6-dihydroxyanthraquinone.

4. Use according to Claim 3, in which the virus is a herpes simplex virus (HSV), cytomegalovirus (CMV) or a human herpes type 6 virus (HHV 6).

## Revendications

1. Utilisation d'un composé de formule générale (I) pour la fabrication d'un médicament pour la prophylaxie et/ou le traitement de maladies provoquées par un virus de la famille des herpétoviridés

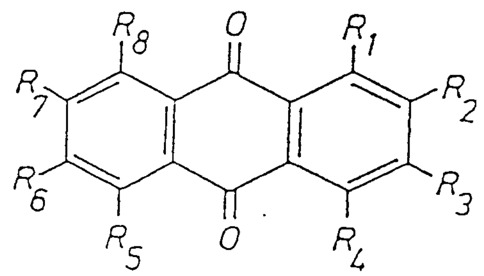

I

dans laquelle
$R_1$ à $R_8$ représentent H, OH, alkyle en $C_1$, alkyle en $C_2$, alkyle en $C_3$, alkyle en $C_4$, $NH_2$, COOH, $SO_3$, $CH_2OH$ ou $C_6H_{11}O_5$, à condition que les résidus $R_1$ à $R_8$ ne soient pas tous indentiques et leurs sels, esters et éthers pharmaceutiquement acceptables, à l'exception de l'aloe-émodine et d'autres extraits des plantes contenant des anthraquinones obtenus à partir de Aloe vera, Rhamnus frangula, Rhamnus purshiana, Cassia angustifolia et Rheum rhaponticum, ainsi que des 2,6-ou 2,7-bis-alkyl (éventuellement halogéné)-aminoalkoxyanthraquinones et leurs sels d'addition acides.

**2.** Utilisation selon la revendication 1 avec au moins un composé, pour lequel les résidus $R_1$ à $R_8$ dans la formule générale (I) représentent :

| | |
|---|---|
| $R_1$ | : H, OH, $CH_3$, $NH_2$ |
| $R_2$ | : H, OH, $CH_3$, $CH_2OH$, COOH, $C_2H_5$, $NH_2$, $SO_3$ |
| $R_3$ | : H, OH, $CH_3$ |
| $R_4$ | : H, OH, $NH_2$ |
| $R_5$ | : H, OH, $HN_2$ |
| $R_6$ | : H, OH, $CH_3$ |
| $R_7$ | : H, OH, $C_6H_{11}O_5$ et |
| $R_8$ | : H, OH, $NH_2$. |

**3.** Utilisation selon la revendication 1 ou 2 avec au moins un des composés suivants : 1,2-dihydroxy-9,10-anthracènedione, acide 9,10-dihydro-3,4-dihydroxy-9,10-dioxo-2-anthracènesulfonique. sel monosodique, 1,4-dihydroxyanthraquinone, 1,8-dihydroxyanthraquinone et 2,6-dihydroxyanthraquinone.

**4.** Utilisation selon la revendication 3, dans laquelle le virus est un virus Herpes simplex (HSV), cytomégalovirus (CMV) ou un herpèsvirus de type 6 (HHV 6).